# EUROPEAN PATENT SPECIFICATION

(11) **EP 1 543 828 B1**
(45) Date of publication and mention of the grant of the patent: **25.10.2006**
(21) Application number: 04257884.9
(22) Date of filing: 16.12.2004
(51) Int. Cl.: A61Q 19/00, A61K 8/02, A61K 8/73, A61K 9/70

(54) **Gel sheet and preparation method thereof**
Gel-Folie und Verfahren zu ihrer Herstellung
Feuille de gel et son procédé de préparation

(30) Priority: 19.12.2003 JP 2003421969
(43) Date of publication of application: 22.06.2005
(73) Proprietor: Shin-Etsu Chemical Co., Ltd., Chiyoda-ku Tokyo 100 (JP)
(72) Inventor: Fukasawa, Miyuki, c/o Shin-Etsu Chem. Co. Ltd., Naka Kubiki-gun, Niigata-ken (JP); Hayakawa, Kazuhisa, c/o Shin-Etsu Chem. Co. Ltd., Naka Kubiki-gun, Niigata-ken (JP)
(74) Representative: Tanner, James Percival

(56) References cited:
- EP-A- 0 993 936
- EP-A- 1 437 216
- WO-A-01/01950
- WO-A-01/78678
- PATENT ABSTRACTS OF JAPAN vol. 2003, no. 12, 5 December 2003 (2003-12-05) & JP 2003 300852 A (SHIN ETSU CHEM CO LTD), 21 October 2003 (2003-10-21)

## Description

### BACKGROUND OF THE INVENTION

### 1. Field of the Invention

The present invention relates to a gel sheet used for various purposes such as cosmetics and pharmaceuticals, and a preparation method of the gel sheet.

### 2. Description of the related art

Gel sheets comprising mainly of agar are conventionally known. Since the agar as a gel forming component forms a network structure in water, such gel sheets can be used for the purpose of moisture retention. However, the gel sheets comprising agar do not have a sufficient strength.

Gel sheets of an acrylic polymer are not free from the fear of toxicity of the residual monomer and gel sheets of other polymers need a crosslinking agent, the use of which complicates their preparation process.

Some cosmetic sheets applicable to the skin such as face or hands contain therein a humectant and a polymer for supporting the humectant, while some cosmetic sheets are coated with a humectant-containing component. For example, a cosmetic moisturizing sheet having a humectant such as hyaluronic acid or collagen supported on a fiber sheet is disclosed in Japanese Patent Application Unexamined Publication No. 9-238738/1997. A cosmetic pack comprising chitosan and a medium paste is disclosed in Japanese Patent Application Unexamined Publication No. 6-48917/1994 and a collagen-free moisturizing mask obtained by crosslinking a cationic biopolymer is disclosed in Unexamined Japanese Publication No. 2001-502678, which is the Japanese language national phase publication of International Patent Application No. PCT/EP97/05618.

The cosmetic sheets, the pack and the mask are ordinarily obtained by impregnating a paper sheet with a humectant. They do not have sufficient water retention capacity and in addition, the paper sheet causes a sense of discomfort upon use. Gel sheets comprising a polymer gel are accompanied with the drawback that use of a crosslinking agent complicates their preparation process.

As a countermeasure against the above problems, a gel sheet comprising cellulose ether having a low molar substitution degree is disclosed in Japanese Patent Application Unexamined Publication No. 2003-300852. The gel sheet is free from the problem of a monomer which occurs upon use of an acrylic polymer, can be prepared without extra effort because a crosslinking agent is not necessary, has a sufficient water retaining capacity, and does not cause any sense of discomfort upon use. When such a sheet is applied to the body, however, it peels from the body before an active agent such as a humectant, an anti-wrinkle agent, an anti-spot agent or a whitening cosmetic in which the sheet has been impregnated starts to work (20 minutes). Thus, it cannot keep its adhesion for at least 60 minutes, which is minimum time required for the effective delivery of the chemical.

### SUMMARY OF THE INVENTION

With the foregoing in view, the present invention has been completed. An aim of the present invention is to provide a gel sheet having a high water content, having high strength, comprising a polymer harmless to the body and being highly adhesive to the skin.

The present inventors have carried out an extensive investigation with a view to attaining the above-described aim. As a result, it has been found that a gel sheet comprising low-substituted cellulose ether having a molar substitution degree of from 0.05 to 1.0, water-soluble cellulose ether having a molar substitution degree of from 1.1 to 1.4 and water can be prepared readily compared with the preparation using a crosslinking agent, has sufficient water retaining capacity, does not cause any sense of discomfort which will otherwise occur when a paper sheet is used, and has improved adhesion to the body, leading to the completion of the invention.

In the present invention, there is thus provided a gel sheet comprising low-substituted cellulose ether having a molar substitution degree of from 0.05 to 1.0, water-soluble cellulose ether having a molar substitution degree of from 1.1 to 1.4 and water. In the present invention, there is also provided a method for preparing a gel sheet comprising the steps of casting an aqueous alkali solution comprising low-substituted cellulose ether having a molar substitution degree of from 0.05 to 1.0 and water-soluble cellulose ether having a molar substitution degree of from 1.1 to 1.4 on a plate, coagulating the cast solution, and then washing the cast coagulated solution.

The gel sheet according to the present invention has adequate strength and elasticity while having a high water content, and is improved further in the touch feeling and adhesion when applied to the skin.

Therefore, by practice of the present invention there may be provided a gel sheet comprising a polymer, having a high water content and being harmless to the body. There may also be provided a cosmetic moisturizing sheet providing comfortable touch feeling to the skin and exhibiting good adhesion to the skin.

### DETAILED DESCRIPTION OF THE PREFERRED EMBODIMENTS

The present invention will next be described more specifically.

The low-substituted cellulose ether used in the present invention is insoluble in water but soluble in an alkaline solution. Cellulose is generally insoluble in water, but when the hydrogen atom of the hydroxyl group on the glucose ring constituting cellulose is substituted with an alkyl or hydroxyalkyl group, it acquires water solubility, though depending on the degree of its substitution. When the substitution degree is low, however, water solubility is not observed, and instead, such cellulose tends to become soluble in an alkaline solution. In many cases, low-substituted cellulose ether powder is partially swelled with water when it is dispersed in water. When the molar substitution degree is high, it becomes water-soluble and loses alkaline solubility.

The low-substituted cellulose ether to be used in the present invention which is insoluble in water but soluble in an alkali, has a molar substitution degree of from 0.05 to 1.0. Examples of various kinds of low-substituted cellulose ether having a desirable substituent content (the number in parentheses means a molar substitution) include low-substituted methyl cellulose having a methoxyl content of from 3 to 15 wt% (from 0.16 to 0.85), low-substituted hydroxyethyl cellulose having a hydroxyethoxyl content of from 3 to 15 wt% (from 0.08 to 0.45), low-substituted hydroxypropyl cellulose having a hydroxypropoxyl content of from 4 to 20 wt% (from 0.09 to 0.51), and low-substituted hydroxypropyl methyl cellulose having a methoxy content of from 3 to 12 wt% and a hydroxypropoxyl content of from 4 to 20 wt% (from 0.25 to 1.0 in total). The substitution degree of cellulose ether can be measured in accordance with the Japanese Pharmacopoeia.

Such low-substituted cellulose ether is insoluble in water but soluble in an aqueous alkaline solution. It absorbs water and swells therewith. The typical example thereof includes low-substituted hydroxypropyl cellulose commercially available now from Shin-Etsu Chemical Co., Ltd. under the product name of L-HPC. It is listed in the Japanese Pharmacopoeia and widely used as a disintegrant to be incorporated in tablets, particularly in the field of pharmaceutical materials.

A preparation method of such low-substituted cellulose ether is known. For example, as described in Japanese Patent Application Examined Publication No. 57-53100/1982, preparation of alkaline cellulose is required first. The alkaline cellulose is prepared by immersing a pulp sheet, which is a starting material, in an aqueous alkaline solution such as sodium hydroxide, or by mixing pulverized pulp directly with an alkaline solution, or by adding an alkali to a dispersion of pulp powder in an organic solvent. The alkali cellulose thus obtained is placed in a reactor, followed by the addition of an etherifying agent such as propylene oxide or ethylene oxide. The mixture is then thermally reacted to produce cellulose ether. After completion of the reaction, the resulting crude cellulose ether is transferred into another tank, where the alkali is neutralized with an acid. The solid thus obtained is washed, dried and then pulverized, whereby the final product is obtained as a powder. Alternatively, the final product may be prepared by completely or partially dissolving the crude cellulose ether just after the reaction in water, neutralizing the resulting solution, collecting the precipitated polymer, and washing, drying and pulverizing the polymer.

According to the present invention, the method for preparing a gel sheet may comprise steps of dissolving low-substituted cellulose ether in an aqueous alkali solution, mixing the resulting solution with an aqueous solution of a water-soluble substituted cellulose ether, casting the resulting mixture on a plate, coagulating it by neutralization with an acid, and then washing the coagulation.

A similar result may be obtained by dissolving the low-substituted cellulose ether powder which is the final product obtained by the above-described process, in an aqueous alkaline solution, or by dissolving in water the alkali-containing crude cellulose just after the reaction. In the latter case, the crude cellulose ether contains an alkali so that only water may be added as the solvent, but an alkali may be also added to ensure complete dissolution. Any one of the above-described methods is applicable to the present invention.

Examples of the alkali to be added to promote dissolution may include potassium hydroxide and sodium hydroxide. The concentration of the alkali can be selected appropriately because it varies depending on the kind or substitution degree of the substituent for the cellulose ether. It may be preferably from 2 to 25 wt%, particularly preferably from 3 to 15 wt%. As a typical example, a 10 wt% sodium hydroxide solution can be used for low-substituted hydroxypropyl cellulose having a molar substitution of 0.2. The resulting solution is transparent in some cases and is not completely transparent in the other cases, depending on the difference in the distribution of the substituent. Even if the solution is not completely transparent, it is regarded as a solution when the viscosity shows an apparent rise.

The concentration of low-substituted cellulose ether which is insoluble in water but soluble in an alkali, in a solution to be cast, may be preferably from 1 to 40 wt%, especially preferably from 5 to 20 wt%. Concentrations below the range may deteriorate the shape retention property. Concentrations above the range, on the other hand, may disturb uniform dissolution owing to an excessive rise in the viscosity. For dissolution, a conventional stirring apparatus may be used, but a kneader capable of mixing even a highly viscous substance is desired. When a large amount of air bubbles appears in the solution as a result of dissolution, it may be preferable to remove the air bubbles by centrifugal separation or to employ a vacuum stirring apparatus.

The water-soluble cellulose ether to be used in the present invention has a molar substitution degree of from 1.1 to 1.4. In the case of cellulose ether having two or more kinds of substituents such as hydroxypropylmethyl cellulose, this molar substitution degree means a total molar substitution degree of two or more kinds of substituents. At a molar substitution below the range, water-soluble cellulose ether may lose their water solubility. At a molar substitution exceeding the range, on the other hand, the cellulose may become insoluble in an alkali and precipitate when the cellulose is mixed with the alkali solution of the low-substituted cellulose ether.

Examples of the water-soluble substituted cellulose ether may include methyl cellulose, hydroxyethyl cellulose, hydroxypropyl cellulose, hydroxypropylmethyl cellulose, hydroxypropylhydroxyethyl cellulose, hydroxyethylmethyl cellulose, ethyl cellulose, hydroxyethylethyl cellulose and carboxymethyl cellulose.

A preparation method of these kinds of cellulose ether is described, for example, in Japanese Patent Application Unexamined Publication No. 10-158302/1998. Similar to cellulose ether which is insoluble in water but soluble in an alkali, preparation of alkali cellulose is required first. The alkali cellulose may be prepared by immersing a pulp sheet which is a starting material, in an aqueous alkaline solution such as sodium hydroxide, or by mixing pulverized pulp directly with an alkaline solution, or by adding an alkali to a dispersion of pulp powder in an organic solvent. The alkali cellulose thus prepared may be placed in a reactor, followed by the addition of an etherifying agent such as propylene oxide or ethylene oxide. The mixture may be thermally reacted to produce cellulose ether. After completion of the reaction, the resulting crude cellulose ether may be transferred into another tank. When it is a substance sparingly soluble in hot water such as methyl cellulose, it may be subjected to neutralization washing in hot water. When it is a substance soluble in both hot water and cool water such as carboxymethyl cellulose, it is subjected to necessary neutralization, followed by washing with a poor solvent such as water-containing methanol, drying and pulverizing to obtain a final powder product.

According to the present invention, provided is a method for preparing a gel sheet comprising steps of casting an aqueous alkali solution comprising low-substituted cellulose ether having a molar substitution degree of from 0.05 to 1.0 and water-soluble cellulose ether having a molar substitution degree of from 1.1 to 1.4, on a plate, coagulating the cast solution, and then washing the cast coagulated solution. Preferably, an aqueous alkali solution of low-substituted cellulose ether having a molar substitution degree of from 0.05 to 1.0 may be mixed with an aqueous solution of cellulose ether having a molar substitution degree of from 1.1 to 1.4. The concentration of the cellulose ether having a molar substitution degree of from 1.1 to 1.4 in the aqueous solution may be preferably from 1.0 to 10.0 wt%.

An amount of the water-soluble cellulose ether may be preferably from 1 to 50 parts by weight, more preferably from 10 to 30 parts by weight based on 100 parts by weight of the low-substituted cellulose ether insoluble in water but soluble in an alkali. At the amount exceeding the range, when the resulting gel sheet is applied to the skin, the water-soluble cellulose ether may form a film on the skin and a part of the film remains white even after peeling. Thus, the sheet may be a cosmetic moisturizing sheet with inferior feeling upon use. At the amount below the range, on the other hand, the resulting gel sheet may be poor in water retention and adhesion to the body.

No particular limitation is imposed on the stirring method adopted for mixing the aqueous alkali solution of the low-substituted cellulose ether having a molar substitution degree of from 0.05 to 1.0 with the aqueous solution of the cellulose ether having a molar substitution degree of from 1.1 to 1.4. Although any conventional stirring method can be used, a stirrer capable of mixing even a highly viscous substance such as kneader is preferred. When a large amount of air bubbles exists in the solution, it may be preferable to remove the air bubbles by centrifugal separation or to use a vacuum stirring apparatus.

The casting method of the solution is not particularly limited and conventional method can be adopted. The material of the support plate is not particularly limited insofar as it is inert to an alkali or acid, including glass and Teflon (trade mark). For example, the solution may be cast on a glass plate by a casting blade to give an adequate thickness. The thickness may usually range from about 0.1 to 10 mm. When the solution is coagulated in the subsequent step, however, its thickness may be adjusted depending on the application purpose because the thickness of the solution upon casting becomes the thickness of a gel sheet.

The cast solution may be then coagulated by neutralization with an acid. Examples of the acid can include hydrochloric acid, sulfuric acid, acetic acid and citric acid. Its concentration is not particularly limited, but may be preferably from 5 to 30 wt%. Diluted hydrochloric acid having a concentration of 10 wt% may be a typical example of the acid.

The solution can also be coagulated with an ammonium sulfate solution instead of the acid.

The neutralization may be carried out by immersing the support plate with the cast solution in a bath containing an acid solution, or by directly pouring an acid solution to the cast solution with a pipette.

The solution cast on the plate may be placed in a neutralizing tank and after a while, the solution coagulates and becomes a gel sheet. The sheet may be then separated from the support plate, followed by washing with water for about 1 minute. Too short washing may fail to remove the acid adhered to the surface or salt remaining in the gel film, while too long washing may cause leakage of the water-soluble cellulose ether from the film.

According to the present invention, the water content of the gel sheet may be preferably 50 wt% or greater, more preferably from 65 to 99 wt% based on the whole weight of the gel sheet. By using the above method, the water content automatically may fall within a preferable range, but the water content can be adjusted to a more preferable range by drying.

According to the present invention, as a cosmetic or pharmaceutical sheet, the gel sheet may comprise an anti-wrinkle agent such as retinol or an anti-spot agent such as cysteine. The gel sheet may comprise a whitening cosmetic including a humectant such as glycerin, hyaluronic acid, collagen, saccharide, amino acid, placenta extract, sorbitol or polyethylene glycol; a softener such as olive oil, cetyl alcohol, lanolin or stearyl alcohol; a blood circulation promoter such as vitamin E; an anti-inflammatory such as glycyrrhizinic acid; and a skin beautifying agent such as various vitamin Cs. If necessary, a water-soluble organic solvent such as alcohol may be added. Such an agent is added to the alkali solution upon its preparation in advance or by impregnating the sheet in the agent. The content of each of the humectant and organic solvent may be preferably from 5 to 49 wt%.

The present invention will hereinafter be described by Examples.

### Example 1

In 91 g of an aqueous 10 wt% NaOH solution was dissolved 9 g of low-substituted hydroxypropyl cellulose powder (product of Shin-Etsu Chemical Co., Ltd.; molar substitution degree of 0.2) by using a kneader. The resulting solution was centrifuged at 5,000 rpm for 10 minutes to remove bubbles therefrom, whereby a transparent viscous solution was obtained. On the other hand, 2.5 g of hydroxypropylmethyl cellulose powder (product of Shin-Etsu Chemical, Co., Ltd.; a molar substitution degree of 1.15 for the methoxyl group; a molar substitution degree of 0.12 for the hydroxypropoxyl group) was dispersed in 47.5 g of hot water, followed by cooling, whereby a transparent viscous liquid was obtained. These two solutions were mixed and stirred carefully so as not to generate air bubbles, whereby a uniform solution was prepared. A portion of the resulting solution was placed on a glass plate and cast to form a film having a thickness of 0.7 mm by using a doctor's blade. The glass plate was immersed in a vat containing 10 wt% of hydrochloric acid and left still for 3 minutes. The gel sheet thus obtained was peeled from the glass plate and washed for 1 minute with running water in a vat filled with water. The surface of the sheet was wiped with gauze, whereby the gel sheet as a final product was obtained.

The water content of this gel sheet was measured by a drying method and found to be 90 wt%. The sheet was almost transparent and had elasticity.

The sheet provided good touch feeling when adhered to the skin. When it was peeled ninety minutes after the adhesion, the skin did not lose its moistness.

The sheet was cut into a piece of 40 mm × 40 mm and adhered to the skin. It took 90 minutes for the sheet to naturally come off from the skin, suggesting that it exhibited good adhesion.

### Examples 2 to 4

In a similar manner to Example 1 except for use of low-substituted methyl cellulose (a molar substitution degree of 0.28 for the methoxyl group), low-substituted hydroxypropylmethyl cellulose (a molar substitution of 0.13 for the methoxyl group, a molar substitution of 0.18 for the hydroxypropoxyl group) and low-substituted hydroxyethyl cellulose (a molar substitution of 0.2 for the hydroxyethoxyl group) instead of the low-substituted hydroxypropyl cellulose, respectively, gel sheets were prepared.

The water contents of the gel sheets were measured by a drying method and found to be from 80 to 90 wt%. These sheets were semi-transparent or transparent and had elasticity.

The sheets were each cut into a piece of 40 mm × 40 mm and adhered to the skin. It provided good touch feeling. When it was peeled one hour after adhesion, the skin did not lose its moistness.

### Example 5

In 91 g of an aqueous 10 wt% NaOH solution was dissolved 9 g of low-substituted hydroxypropyl cellulose powder (product of Shin-Etsu Chemical Co., Ltd.; a molar substitution degree of 0.2) by using a kneader. The resulting solution was centrifuged at 5,000 rpm for 10 minutes to remove air bubbles therefrom, whereby a transparent viscous solution was obtained. On the other hand, 2.5 g of methyl cellulose powder (product of Shin-Etsu Chemical Co., Ltd.; a molar substitution degree of 1.2 for the methoxyl group) was dispersed in 47.5 g of hot water, followed by cooling, whereby a transparent viscous solution was obtained. These two solutions were mixed and stirred carefully so as not to generate air bubbles, whereby a uniform solution was prepared. A portion of the resulting solution was placed on a glass plate and cast to form a film having a thickness of 0.7 mm by using a doctor's blade. The glass plate was immersed in a vat containing 10 wt% hydrochloric acid and left still for 3 minutes.

The gel sheet thus obtained was peeled from the glass plate and washed for 1 minute with running water in a vat filled with water. The surface of the sheet was wiped with gauze, whereby the gel sheet as a final product was obtained.

The gel sheet thus obtained was immersed in an aqueous 25 wt% glycerin solution for 30 seconds to prepare a gel sheet having 6 wt% of glycerin impregnated. The water content was measured by a drying method and found to be 90 wt%. The sheet was almost transparent and had elasticity.

The sheet provided good touch feeling when adhered to the skin. When it was peeled sixty minutes after the adhesion, the skin remained moister than that of Example 1.

The sheet was cut into a piece of 40 mm × 40 mm and adhered to the skin. It took 90 minutes for the sheet to naturally come off, suggesting that the sheet exhibited good adhesion.

### Comparative Example 1

In 91 g of an aqueous 10 wt% NaOH solution was dissolved 9 g of low-substituted hydroxypropyl cellulose powder (product of Shin-Etsu Chemical Co., Ltd.; a molar substitution degree of 0.2) by using a kneader. The resulting solution was centrifuged at 5,000 rpm for 10 minutes to remove air bubbles, whereby a transparent viscous solution was obtained. A portion of the resulting solution was placed on a glass plate and cast using a doctor's blade to form a film having a thickness of 0.7 mm. The glass plate was immersed in a vat filled with 10 wt% of hydrochloric acid and left still for 3 minutes.

The gel sheet thus obtained was peeled from the glass plate and washed for 1 minute with running water in a vat filled with water. The surface of the sheet was wiped with gauze, whereby a gel sheet as a final product was obtained.

The water content of the gel sheet was measured by a drying method and found to be 90 wt%. The sheet was almost transparent and had elasticity.

The sheet was cut into a piece of 40 mm × 40 mm and adhered to the skin. It naturally peeled from the skin by itself after 20 minutes.

## Claims

1. A gel sheet comprising:
low-substituted cellulose ether having a molar substitution degree of from 0.05 to 1.0,
water-soluble cellulose ether having a molar substitution degree of from 1.1 to 1.4, and
water.

2. The gel sheet according to Claim 1 wherein said water is comprised in an amount of 50% by weight or greater based on the whole weight.

3. The gel sheet according to Claim 1 or 2 wherein said water-soluble cellulose ether is hydroxypropylmethyl cellulose.

4. The use, as a cosmetic sheet, of a gel sheet according to any one of claims 1 to 3.

5. A method for preparing a gel sheet, comprising the steps of:
casting an aqueous alkaline solution comprising low-substituted cellulose ether having a molar substitution degree of from 0.05 to 1.0 and water-soluble cellulose ether having a molar substitution degree of from 1.1 to 1.4 on a plate,
coagulating the cast solution, and
washing the cast coagulated solution.

## Patentansprüche

1. Gelfolie, welche folgendes umfaßt:
niedrig substituierten Zelluloseether mit einem molaren Substitutionsgrad von 0,05 bis 1,0,
wasserlöslichen Zelluloseether mit einem molaren Substitutionsgrad von 1,1 bis 1,4 und Wasser.

2. Gelfolie nach Anspruch 1, wobei das Wasser in einer Menge von 50 Gew.-% oder mehr, basierend auf dem Gesamtgewicht, enthalten ist.

3. Gelfolie nach Anspruch 1 oder 2, wobei der wasserlösliche Zelluloseether Hydroxypropylmethylzellulose ist.

4. Verwendung einer Gelfolie nach einem der Ansprüche 1 bis 3 als kosmetische Folie.

5. Verfahren zur Herstellung einer Gelfolie, welches die folgenden Stufen umfaßt:
Gießen einer wäßrigen alkalischen Lösung, welche niedrig substituierten Zelluloseether mit einem molaren Substitutionsgrad von 0,05 bis 1,0 und wasserlöslichen Zelluloseether mit einem molaren Substitutionsgrad von 1,1 bis 1,4 umfaßt, auf eine Platte,
Koagulieren der gegossenen Lösung und
Waschen der gegossenen koagulierten Lösung.

## Revendications

1. Feuille de gel comprenant :
un éther de cellulose faiblement substitué, ayant un degré de substitution molaire de 0,05 à 1,0,
un éther de cellulose hydrosoluble ayant un degré de substitution molaire de 1,1 à 1,4, et
de l'eau.

2. Feuille de gel selon la revendication 1, dans laquelle ladite eau est contenue en une proportion de 50 % en poids ou plus par rapport au poids total.

3. Feuille de gel selon la revendication 1 ou 2, dans laquelle ledit éther de cellulose hydrosoluble est de l'hydroxypropylméthylcellulose.

4. Utilisation, comme feuille cosmétique, d'une feuille de gel selon l'une quelconque des revendications 1 à 3.

5. Procédé de préparation d'une feuille de gel, comprenant les étapes de :
coulée d'une solution aqueuse alcaline comprenant un éther de cellulose faiblement substitué ayant un degré de substitution molaire de 0,05 à 1,0 et un éther de cellulose hydrosoluble ayant un degré de substitution molaire de 1,1 à 1,4, sur une plaque,
coagulation de la solution coulée, et
lavage de la solution coulée coagulée.
